# EUROPEAN PATENT SPECIFICATION

(11) **EP 1 843 664 B1**
(45) Date of publication and mention of the grant of the patent: **08.07.2009**
(21) Application number: 04803365.8
(22) Date of filing: 30.11.2004
(51) Int. Cl.: A23B 7/155, A23L 1/03, A23L 1/212, A23L 1/30, C12N 1/20

(54) **TABLE OLIVES CONTAINING PROBIOTIC MICROORGANISMS**
TAFELOLIVEN, DIE PROBIOTISCHE MIKROORGANISMEN ENTHALTEN
OLIVES DE TABLE CONTENANT DES MICRO-ORGANISMES PROBIOTIQUES

(30) Priority: 05.12.2003 IT MI20032391
(43) Date of publication of application: 17.10.2007
(73) Proprietor: CONSIGLIO NAZIONALE DELLE RICERCHE, 00185 Roma (IT)
(72) Inventor: LAVERMICOCCA, Paola, 70126 Bari (IT); LONIGRO, Stella, Lisa, 70126 Bari (IT); VISCONTI, Angelo, 70126 Bari (IT); DE ANGELIS, Maria, 70126 Bari (IT); VALERIO, Francesca, 70126 Bari (IT); MORELLI, Lorenzo Universita del Sacro Cuore, 29100 Piacenza (IT)
(74) Representative: Nemni, Raffaelo
(86) International application number: PCT/EP2004/013582
(87) International publication number: WO 2005/053430

(56) References cited:
- EP-A- 1 308 506
- WO-A-00/60948
- WO-A-97/49303
- WO-A-02/056695
- US-A- 3 891 771
- US-A- 5 603 930
- VAN DEN BERG DJC, ET AL: "Isolation, screening and identification of lactic acid bacteria from traditional food fermentation processes and culture collections" FOOD BIOTECHNOLOGY, vol. 7, no. 3, 1993, pages 189-205, XP009046122
- SUSKOVIC J ET AL: "Probiotic properties of Lactobacillus plantarum L4" FOOD TECHNOL. BIOTECHNOL., vol. 35, no. 2, 1997, pages 107-112, XP009046128

## Description

### Field of the invention

The present invention relates to probiotic food products, i.e. food products containing microorganisms having a beneficial effect on health, in particular on the gastrointestinal tract.

### Background of the invention

Probiotic food products are in general fermented foods containing an amount of viable and active microorganisms large enough to reach the intestine and exert an equilibrating action on the intestinal microflora.

Intake of probiotics stimulates the growth of beneficial microorganisms, reduces the amount of pathogens and strengthens the body's natural defences. It is acknowledged that probiotic bacteria, in particular lactobacilli and bifidobacteria, help to maintain the equilibrium of the intestinal flora (Salminen S., et al. Int. Dairy J. 8:563-572, 1998; Saarela M., L. et al., Int. J. Food Microbiol. 2002, 78:99-117) and inhibit pathogens (Drago L., M. R.et al., FEMS Microbiol. Letters, 1997, 153:455-463 and Cross M. L. FEMS Immunol. Med. Microbiol. 2002, 34:245-253), thus lowering the risk of gastro-intestinal diseases. In fact, when the intestinal microflora is altered, administration of probiotic bacteria not only re-establishes its normal equilibrium, but also improves the microbial balance and properties of the endogenous flora. The role of probiotics in the prevention of food allergies and intolerances is also under study (Isolauri E., et al., Am. J. Clin. Nutr. 2001, 73 (suppl.): 444s-450s; Jahreis G., et al. Food Res. Int. 2002, 35:133-138).

Probiotic bacteria are introduced in food products for human nutrition, especially in fermented milk, for example in yogurt. One of the problems related to the production of probiotic foods is the influence of production technologies on strains properties, in particular cell viability, integrity, and population stability (Mattila - Sundholm T., et al. Int. Dairy, 2002 J. 12:173-182). Liquid and frozen cultures where largely used in the past, but their production, transport and storage costs are high. Lypohilised cultures are presently widespread, but cells are often damaged and cannot be stored for a long time. In fact lyophilised cells survive in anaerobiosis and viability is restored by rehydratation. This treatment not only does not insure survival of all cells but the survived ones may also be methabolically altered and not withstand gastric acidity. Concentraded monodose cell cultures are also widespread. In this case the greatest difficulty is to reach high cell concentrations, i.e. to about 10¹⁰ (UFC) / g. Therefore, most of the presently available probiotics are of animal origin, in particular dairy products, such as yogurt, cheese, deserts, ice cream. However, dairy product consumption may be limited due to allergies or intolerance to milk and derivatives thereof. Also known is the difficulty of introducing befedobacteria - largely used in probiotics in fermented milk products, due to their strain related sensibility to milk - fermenting bacteria, pH, temperature and oxygen concentration (Gobbetti M. et al. J. Dairy She. 1998, 81:37 - 47).

Probiotic dehydrated fruits have been obtained on an experimental scale by vacuum draying fruits soaked in probiotic microorganism (Betoret N., et al. J. Food Engin. 2003, 56:273 - 277), while some oat based products and fruit juices containing probiotic bacteria are already available on the market (Johansson et al. Int. J. Food Microbiol., 1998,42:29 - 38).

It should also be pointed out that all the above mentioned products must be consumed rapidly after opening.

In relation to vegetables, it is known that *Lactobacillus paracasei* were isolated from olive brains (Van Den Berg DJC et AL., "Isolation, screening and identification of lacto acid bacteria from traditional food fermentation processes and culture collections", Food Biotechnology, vol. 7, no 3, 1993, pages 189-205). It also known from document WO00/60948 a process for fermenting vegetables products applicable to all possible varieties of olives including an additional step of inoculating the brine in which the olives are placed with a culture of micro-organism *Lactobacillus plantarum,* LP RJL1, which produces a bacteriocin called plantaricin S and from WO 02/05695 A a method for fermenting vegetable products applicable gherkins, carrots and all possible varieties of olives, involving the inoculation of the brine, in which the vegetable product are placed, with a mixed culture comprising two micro-organism: *Lactobacillus plantarum,* LP RJL2 and *Lactobacillus plantarum* LP RJL3.

It should therefore be advantageous to provide food products, in particular olives, that allow to administer probiotic bacteria without causing allergies or intolerances and that can be stored for a long time after opening.

The present invention is defined by the claims and relates to probiotic food products based on table olives containing probiotic bacteria.

In a first embodiment, the food product consists of table olives whose pericarp is coated with microorganisms of the *Lactobacillus* and *Bifidobacterium* genus, in particular probiotic lactobacilli and bifidobacteria. Preferably, the lactobacilli are selected from *Lactobacillus rhamnosus* and *Lactobacillus paracasei,* while the bifidobacteria are selected from *Bifidobacterium bifidum* and *Bifidobacterium longum.* Even more preferably, the microorganisms are selected from: *Lactobacillus rhamnosus* GG ATCC53103; *L. rhamnosus* IMPC 11; *L. rhamnosus* IMPC 19; *Lactobacillus paracasei* IMPC 2.1 (deposited with the Belgian Coordinated Collections of Microorganisms, BCCMILMG-Collection, Gent, Belgium, under accession number LMG P-22043); *Lactobacillus paracasei* IMPC 4.1; *Bifidobacterium bifidum* ATCC15696 and *Bifidobacterium longum* ATCC15708.

The olives of the invention can be prepared by keeping table olives in a suspension of the desired microorganism, at room temperature (about 25°C), thus obtaining olives on whose pericarp microorganisms adhere in amounts ranging from 5x10⁵ to 5x10⁸ UFC/gram (evaluation after 3 month storage, see tables 1 and 2).

The table olives of the invention can be either consumed as such, or used for the preparation of probiotic food products, which are a further embodiment of the invention.

The olives and probiotic foods of the invention are an effective means to treat or prevent intestinal disorders or restore the intestinal flora after antibiotic therapy.

Particularly beneficial are the olives enriched with *L. paracasei* IMPC 2.1, not only due to the marked probiotic characteristics of this microorganism, its ability to grow both under aerobic and anaerobic conditions and adhere to the pericarp, but also due to its resistance to gastric juices and bile salts. *L. paracasei* IMPC 2.1 is a new microorganism and is a further embodiment of the invention.

Particularly important is also the possibility of incorporating bifidobacteria, since it is known that these microorganisms hardly grow and survive in fermented milk products.

The olives of the present invention and the food products containing them are particularly useful for the prevention and treatment of diseases caused by food contaminants, in gastro-intestinal diseases affecting travellers, as coadjuvants in antibiotic therapy and, more generally, in situations in which it is necessary to increase the body immune defences.

Thanks to convenient administration, storage in non-refrigerated conditions (after 90 days at room temperature the bacterial count ranges from 1x10⁵ to 7.6x10⁷ UFC per gram), as well as organoleptic properties, the olives can be consumed whenever prompt administration of probiotic bacteria is required, even by lactose-intolerant people. A further advantage is that consumption of only part of the package content (i.e. olives, not brine), provides a dose of probiotic bacteria that corresponds to that provided by yogurt or concentrated cultures.

Finally, it must also be pointed out that with respect of probiotic foods of animal or vegetal origin, wherein microorganisms are re-suspended in a liquid medium, in the case of olives the bacterial cells are immobilized, which ensures an effective, safe transport in the gastro-intestinal tract. Moreover, the binding to a product containing a large amount of fats, allows the microorganisms to resist to gastric juices.

### EXPERIMENTAL SECTION

### Example 1 - Viability of probiotic bacteria on the olive pericarp

Colonization of the pericarp of table olives and the survival of the following strains have been evaluated: *Lactobacillus rhamnosus* GG ATCC3103, *L. rhamnosus* IMPC 11 and IMPC 19, *Lactobacillus paracasei* IMPC 2.1 and IMPC 4.1, *Bifidobacterium bifidum* ATCC15696 and *Bifidobacterium longum* ATCC15708.

*Lactobacillus paracasei* IMPC 2.1 was deposited with the Belgian Coordinated Collections of Microorganisms, BCCM/LMG-Collection, Gent, Belgio under accession number LMG P-22043.

Tests were carried out on stoned and whole black olives, previously de-bittered and processed so as to make them edible. The same tests were also carried out on fresh or semifinished green and black olives and on de-bittered and processed green olives (finished product). Strains viability was evaluated using jars containing 80 olives immersed in 280 ml of their own brine or in NaCl 4% ± fructose 0.2+1 %, pH 6.5.

*Procedure.* Black olives immersed in their own brine (finished product) were added with a bacterial suspension containing from 4x10⁹ to 9x10¹¹ (UFC) of each strain. After the inoculum the olives are placed in sterile jars closed with screw-caps. Non-inoculated olives, also in jars, were used as the control. The samples were stored for 3 months at room temperature (about 25°C), thereafter 4 olives were taken from each sample at t=1, 15, 30 and 90 and submitted to bacterial count. Brine was thoroughly removed and the olives were added with 20 ml 0.85% NaCl and 0.025% Tween 80 and vigorously shaken for two hours to detach the bacteria from the pericarp. The resulting suspension was seeded on an agar substrate for the count of lactic bacteria. The results are reported in the following table.

**Table 1. UFC per gram of stoned olives**

| strain | 1 day | 15 days | 30 days | 90 days |
|---|---|---|---|---|
| *L. rhamnosus* GG ATCC53103 | 5.0 x 10⁸ | 3.5 x 10⁷ | 3.4 x 10⁷ | 3.2 x 10⁷ |
| *L. rhamnosus* IMPC 11 | 7.0 x 10⁷ | 8.0 x 10⁸ | 7.5 x 10⁷ | 7.6 x 10⁷ |
| *L. rhamnosus* IMPC 19 | 7.2 x 10⁷ | 8.5 x 10⁸ | 6.8 x 10⁶ | 6.9 x 10⁶ |
| *L. paracasei* IMPC 2.1 | 7.6 x 10⁷ | 2.0 x 10⁹ | 7.9 x 10⁷ | 6.0 x 10⁶ |
| *L. paracasei* IMPC 4.1 | 4.8 x 10⁷ | 5.8 x 10⁷ | 6.5 x 10⁷ | 6.8 x 10⁷ |
| *B. bifidum* ATCC 15696 | 2.5 x 10⁷ | 8.0 x 10⁷ | 5.2 x 10⁷ | 4.3 x 10⁶ |
| *B. longum* ATCC 15708 | 4.5 x 10⁶ | 2.7 x 10⁷ | 8.7 x 10⁶ | 5.2 x 10⁵ |

**Table 2. UFC per gram of whole olives**

| strain | 1 day | 15 days | 30 days | 90 days |
|---|---|---|---|---|
| *L. rhamnosus* GG ATCC53103 | 1.8 x 10⁷ | 2.3 x 10⁶ | 7.6 x 10⁵ | 3.9 x 10⁶ |
| *L. rhamnosus* IMPC 11 | 7.0 x 10⁶ | 1.0 x 10⁷ | 2.4 x 10⁶ | 1.5 x 10⁶ |
| *L. rhamnosus* IMPC 19 | 3.5 x 10⁶ | 1.0 x 10⁷ | 2.5 x 10⁵ | 2.7 x 10⁵ |
| *L. paracasei* IMPC 2.1 | 7.4 x 10⁶ | 3.0 x 10⁷ | 4.4 x 10⁷ | 9.0 x 10⁶ |
| *L. paracasei* IMPC 4.1 | 7.1 x 10⁶ | 1.1 x 10⁷ | 5.4 x 10⁷ | 8.0 x 10⁶ |
| *B. bifidum* ATCC 15696 | 1.3 x 10⁶ | 7 x 10⁶ | 3.6 x 10⁶ | 1.2 x 10⁶ |
| *B. longum* ATCC 15708 | 5.0 x 10⁵ | 3.6 x 10⁶ | 3.6 x 10⁶ | 1.0 x 10⁵ |

All the experiments were repeated twice and relevant variations were not observed.

The pericarp allows tight anchorage of the bacteria and ensures their slow release after intake, as demonstrated by the drastic re-suspension procedure. In particular, about 10⁶ UFC/g were recovered from samples analysed 30 days after addition of the bacteria by vigorous stirring for 2 hours in physiological solution added with Tween; after 3 subsequent washings (1 h each in the same conditions) about 10⁵, 10⁴ and 10³ UFC/9 g still adhered to the pericarp.

Similar tenacity was observed in samples taken after 7 or 90 days from the addition of the bacteria.

### Example 2 - Selection of Lactobacillus paracasei IMPC 2.1 (reference strain)

*Lactobacillus paracasei* IMPC 2.1 was isolated from a healthy adult human subject with a bacterial population of 10⁷ UFC/ g in faeces.

### Strain genetic identification

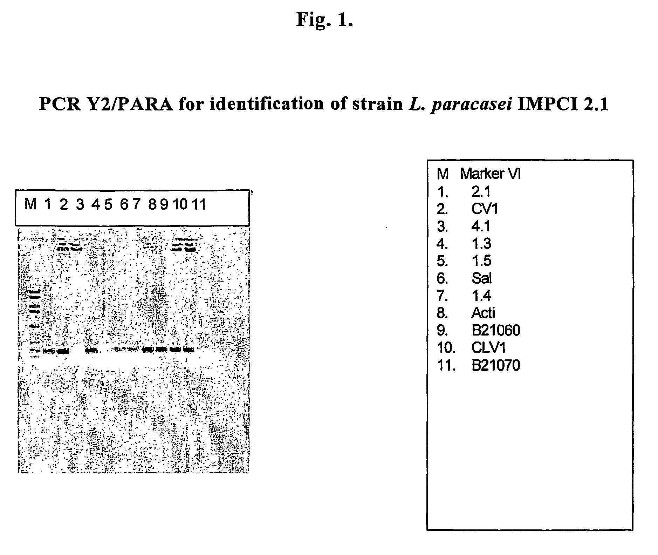

Species-specific PCR with Y2/PARA primers (figure 1) was carried out as the first identification step. Y2 is the universal primer for eubacteria, while PARA is the specific primer for *L. paracasei.* IMPC 2.1 showed an amplification band of 290 bp, typical *of L. paracasei* species.

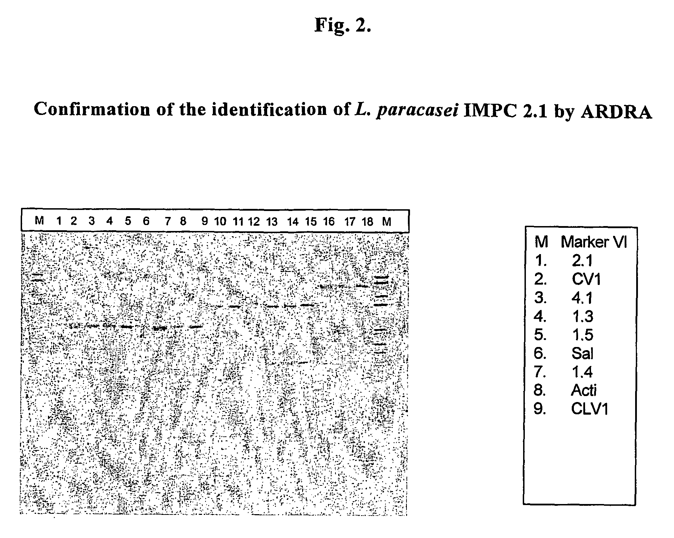

ARDRA using Sau 3AI as the restriction enzyme was carried out as a confirmation analysis; also in this case the expected restriction profiles of *L. paracasei* were obtained (Figure 2).

*L. paracasei* IMPC 2.1 is able to tightly adhere to pig intestinal mucus, abiotic surfaces and pericarp and is highly resistant to bile acids, as demonstrated by the following experiments.

### Adhesion to pig intestinal mucus

An *in vitro* test for adhesion to pig intestinal mucus was carried out to evaluate *in vivo* adhesion, according to the method of Schou, et al. (APMIS 1999, 107: 493-504), partially modified as follows.

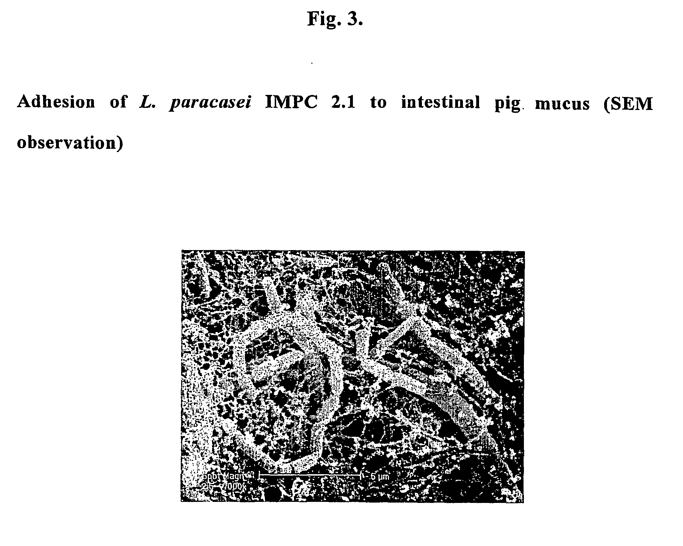

96-Well plates, coated with pig mucus (Tipe II, Sigma), were seeded with a titred bacterial suspension (100 µl, PBS buffer). After incubation for 2 hrs at 37°C with rocking, the plates were washed three times with PBS and the mucus was mechanically removed from the wells, then the washings and mucus were seeded in plates. Figure 3 reports a SEM image *of L. paracasei* IMPC 2.1 adhering to the mucus after three washings.

The *L. paracasei* strains used in the test are listed hereinbelow, together with the results of the count (percentage ratio of UFC on the mucus in the final step to UFC in the titred bacterial suspension)
1) IMPC 2.1= 40%
2) IMPC CV1= 37%
3) IMPC 4.1= 10%
4) IMPC 1.3= 40%
5) IMPC 1.5= 33%
6) IMPC 1.4= 35%
7) Chr.Hansen Lc1= 39%
8) IMPC CLV1= 38%
9) ATCC 10863= 18%

IMPC 2.1 is one of the strains which adhere better.

### Resistance to bile salts

The resistance of *L. paracasei* strains was evaluated using MRS medium (De Man et al., J. Appl. Bacteriol., 1960, 23:130-135) containing Oxgall bovine bile salts at different concentrations. The first tests were carried out using 0.2, 0.3, 0.4% Oxgall: in these conditions the strain showed slightly reduced growth at increased concentrations. Growth was evaluated by measuring optical density (OD) at 600 nm.

| Strains | MRS | 0.2% Oxgall | 0.3% Oxgall | 0.4% Oxgall |
|---|---|---|---|---|
| **2.1** | **1.847** | **1.678** | **1.739** | **1.570** |
| Acti | 1.942 | 1.587 | 1.314 | 1.043 |
| Sal | 1.942 | 1.674 | 1.583 | 1.451 |
| CV 1 | 1.853 | 1.640 | 1.518 | 1.312 |
| CLV 1 | 1.813 | 1.688 | 1.634 | 1.344 |
| B21070 | 1.714 | 1.455 | 1.316 | 1.185 |
| B21060 | 1.954 | 1.789 | 1.657 | 1.453 |
| 1.3 | 1.829 | 1.818 | 1.697 | 1.583 |
| 1.4 | 1.843 | 1.840 | 1.679 | 1.5 81 |
| 1.5 | 1.875 | 1.760 | 1.818 | 1.674 |
| 4.1 | 1.978 | 1.694 | 1.441 | 1.559 |

In the subsequent step (the bile acid) concentration was increased up to 0.7%.

| Strain | MRS | 0.5% Oxgall | 0.6% Oxgall | 0.7% Oxgall |
|---|---|---|---|---|
| **2.1** | **1.458** | **0.792** | **0.178** | **0.095** |
| Acti | 1.548 | 0.139 | 0.061 | -0.132 |
| Sal | 1.354 | 0.758 | 0.562 | 0.353 |
| CV1 | 1.399 | 0.160 | -0.038 | -0.156 |
| CLV1 | 1.313 | 0.322 | 0.176 | 0.055 |
| B21070 | 1.435 | -0.142 | -0.200 | -0.193 |
| B21060 | 1.367 | 0.729 | 0.611 | 0.280 |
| 1.3 | 1.377 | 0.576 | 0.234 | 0.314 |
| 1.4 | 1.525 | 0.695 | 0.927 | 0.396 |
| 1.5 | 1.475 | 0.866 | 0.916 | 0.603 |
| 4.1 | 1.502 | 0.817 | 0.764 | 0.561 |

IMPC 2.1 proved one of the strains with good resistance to bile salts.

### Salinity resistance

MRS medium was used to evaluate strain resistance to different NaCl concentrations. Also in this case growth was evaluated by measuring optical density (OD) at 600 nm.

| Strain | MRS | 0.5% NaCl | 1% NaCl | 2% NaCl |
|---|---|---|---|---|
| **2.1** | **1.847** | **1.644** | **1.457** | **1.513** |
| Acti | 1.942 | 1.551 | 1.689 | 1.483 |
| Sal | 1.942 | 1.685 | 1.665 | 1.601 |
| CV1 | 1.853 | 1.555 | 1.541 | 1.781 |
| CLV1 | 1.813 | 1.512 | 1.689 | 1.648 |
| B21070 | 1.714 | 1.711 | 1.658 | 1.491 |
| B21060 | 1.954 | 1.560 | 1.717 | 1.510 |
| 1.3 | 1.829 | 1.656 | 1.631 | 1.697 |
| 1.4 | 1.843 | 1.658 | 1.795 | 1.737 |
| 1.5 | 1.875 | 1.534 | 1.554 | 1.697 |
| 4.1 | 1.978 | 1.811 | 1.762 | 1.596 |

Since high growth rate was observed also with 2% NaCl, tests with higher concentrations were carried out.

| Strain | MRS | 3% NaCl | 4% NaCl | 5% NaCl |
|---|---|---|---|---|
| **2.1** | **1.300** | **1.217** | **1.081** | **0.896** |
| Acti | 1.327 | 1.317 | 1.169 | 1.073 |
| Sal | 1.288 | 1.275 | 1.180 | 0.985 |
| CV 1 | 1.283 | 1.185 | 1.031 | 0.829 |
| CLV1 | 1.217 | 1.158 | 1.036 | 0.799 |
| B21070 | 1.266 | 1.269 | 1.128 | 0.947 |
| B21060 | 1.321 | 1.177 | 1.090 | 0.962 |
| 1.3 | 1.239 | 1.207 | 1.050 | 0.922 |
| 1.4 | 1.306 | 1.183 | 1.026 | 0.823 |
| 1.5 | 1.289 | 1.266 | 1.061 | 0.899 |
| 4.1 | 1.291 | 1.245 | 1.075 | 0.897 |

IMPC 2.1 proved to be one of the strains with better salinity resistance.

### Resistance to simulated gastric juice (UFC/ml)

Strain resistance to simulated gastric juice was evaluated using different strains cultured in MRS medium. The cultures were washed with sterile saline and added to an equal volume of simulated gastric juice (NaCl, 125mM⁻¹; KCl 7 mM⁻¹; NaHCO₃, 45 mM⁻¹ and pepsin, 3 gr 1⁻¹), adjusting the pH to 2 with HCl. The suspensions were then incubated at room temperature under stirring (200 rev min⁻¹) to simulate peristalsis. Aliquots were taken at time 0 and after 90 and 150 minutes and counted on MRS agar.

| Strain | T₀ | T₁(90 min) | T₂(150 min) |
|---|---|---|---|
| **2.1** | **44•10⁶** | **1.22•10⁸** | **2.15•10⁷** |
| 1.4 | 41•10⁶ | 4.5•10⁷ | 3•10⁷ |
| B21070 | 17.8•10⁶ | 2•0⁷ | 5.8•10⁷ |
| Sal | 197•10⁶ | 1.13•10⁸ | 5.2•10⁷ |
| 4.1 | 116•10⁶ | 1.09•10⁸ | 1.77•10⁷ |

IMPC 2.1 proved one of the strains with better resistance to simulated gastric juice.

### Adhesion to abiotic surfaces

Adhesion ability, necessary for the strains to colonize the intestinal mucosa, was evaluated also with a test for adhesion to abiotic surfaces (Tuomola et al., Int. J. Food Microbiol., 2000, 41:45-51). The strains were cultured in MRS medium, at 37°C for 48 hours under anaerobiosis. The cultures were then diluted 1:40 in MRS and 200 µl aliquots were seeded in 96-well polystyrene plates. After incubation for 24 hours at 37°C the wells were gently rinsed with Dulbecco's phosphate buffer (DPBS, pH 7.3), allowed to dry and added with a crystal violet solution to stain the cells. Excess of dye was washed away with ethanol-acetone (80:20 v/v), then optical density (DO) was measured with an automatic reader. On the basis of DO values, cells were divided into 4 adhesion classes: no adhesion (AC1, OD≤0.5), weak adhesion (AC2, 0.5<OD≤1.2), mean adhesion (AC3, 1.2<OD≤2.0) and strong adhesion (AC4, OD>2.0) (Table 3).

To evaluate the effect of enzymatic, physical and chemical treatment on the adhesion ability of the strains, bacterial cultures at the beginning of the stationary phase (6 hrs growth) were submitted to the said treatments at various temperatures and times, thereafter adhesion changes were evaluated. The adhesion properties are reported in the following table. The results show that the adhesion properties of the strains are generally not much altered by physical, chemical and enzymatic treatment.

**Table 3. Adhesion of L. paracasei IMPC 2.1, compared with L. rhamnosus GG ATCC53103 and another L. paracasei strain, on an abiotic surface before and after physical, chemical and enzymatic treatment.**

| Strain Treatment | *L. rhamnosus* GG ATCC53103 | ***L. paracasei*** **IMPC 2.1** | *L.paracasei* IMPC 4.1 |
|---|---|---|---|
| | | | |
| Cultures incubated for 24 h in wells | 4 | **4** | 2 |
| Control cells (6 hrs incubation) | 4 | **4** | 2 |
| **Physical treatment** | | | |
| 30 min/65°C | 1 | **2** | 1 |
| 15 min/120°C | 1 | **1** | 1 |
| **Enzymatic Treatment** | | | |
| Buffer A | 4 | **3** | 2 |
| 5.0 mg/ml trypsin | 1 | **2** | 1 |
| 5.0 mg/ml proteinase | 1 | **2** | 1 |
| 5.0 mg/ml chemotrypsin | 1 | **2** | 1 |
| Buffer B | 4 | **3** | 2 |
| 5.0 mg/ml pepsin | 2 | **3** | 1 |
| **Chemical Treatment** | | | |
| Buffer C | 4 | **3** | 2 |
| 0.05 M sodium periodate | 4 | **3** | 2 |
| 0.05 M sodium iodate | 4 | **3** | 2 |
| SM LiC1 | 2 | **2** | 1 |

| | | | |
|---|---|---|---|
| ^{a} Adhesion Class (AC):1, OD≤.5; 2, 0.5<OD≤1.2; 3, 1.2<OD≤2.0; 4, OD>2 | | | |

### Adhesion to pericarp

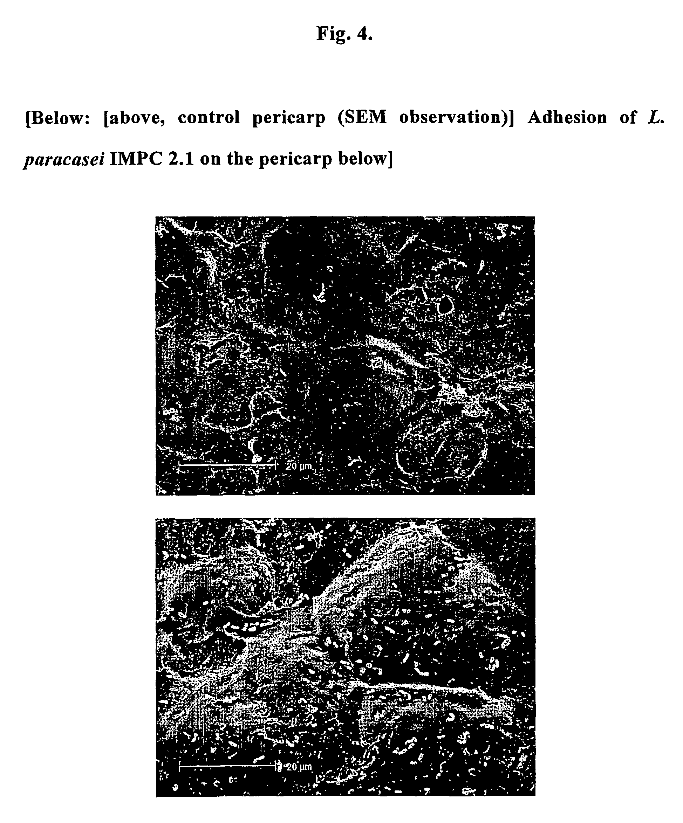

Figure 4 shows anchoring and distribution of *L. paracasei* IMPC 2.1 on the pericarp (see also tables 1 and 2).

### Example 3 - Persistence of L. paracasei IMPC 2.1 in the gastro-intestinal tract

### Experiment 1

Two healthy adult subjects were fed for 7 days with portions of 5 (subject 1) and 10 (subject 2) olives, thoroughly drained, containing in all 3x10¹⁰ and 6x10¹⁰ UFC of *L. paracasei* IMPC 2.1 respectively. The composition of the intestinal flora of the subjects was monitored at the beginning (time 0) and after 7 days (t=7) of administration and after 3 days from the end of administration. At each sampling, 1 g of faeces from each subject was added with 9 ml of Amies medium, homogenized and submitted to decimal dilutions, which were plated on a 12 µg/ml Rogosa ± vancomycin substrate and cultured under anaerobiosis for 48 hours at 37°C.

**Table 4. Lactic populations in human subjects before and after administration of table olives added with L. paracasei IMPC 2.1.**

| | Total UFC on Rogosa + vancomycin | | |
|---|---|---|---|
| | t=0 | t=7 days | t=3 days after suspension |
| Subject 1 fed with 3 x 10¹⁰ UFC/die | 2.7 x 10⁷ | 2 x 10⁹ | 4.5 x 10⁶ |
| Subject 2 fed with 6 x 10¹⁰ UFC/die | 7.0 x 10⁴ | 3.1 x 10⁶ | 5.2 x 10⁵ |

An increase of about two logarithmic cycles in the intestinal lactic population of the two subjects was observed; an expected reduction of about 2.5 cycles in subject 1 and of about 1 cycle in subject 2 was observed after suspension of the administration.

### Experiment 2

Two healthy adult subjects (A and B) were fed with portions of ten olives containing about 10⁹ CFU *of L. paracasei* IMPC 2.1. The intestinal microflora of was monitored at the beginning of the experiment (t=0), after 10 days of daily consumption of the product (t=10) and 7 days from the end of administration, according to the procedure described in experiment 1. The results are reported in the following table.

The colonies isolated in both experiments were subjected to molecular identification (see Example 2), whereby it was ascertained that *L. paracasei* IMPC 2.1 was present in the two subjects and colonized the intestine.

## Claims

1. Table olives containing probiotic lactobacilli adhering on the pericarp selected from *Lactobacillus rhamnosus and L. paracasei* and/or bifidobacteria adhering on the pericarp selected from *Bifidobacterium bifidum and B. longum* **characterized in that** the lactobacilli are selected from: *Lactobacillus rhamnosus* GG ATCC53103; *L. rhamnosus* IMCP 11; *L. rhamnosus* IMCP 19; *Lactobacillus paracasei LMG P 22043; Lactobacillus paracasei* IMPC 4.1 and that the bifidobacteria are selected from *Bifidobacterium bifidum* ATCC 15696 and *Bifidobacterium longum* ATCC15708;

2. Table olives according to claim 1 **characterized in that** the lactobacilli belong to the strain *Lactobacillus paracasei* deposited with the Belgian Coordinated Collections of Microorganism under accession number LMG P 22043;

3. Use of lactobacilli and bifidobacteria to coat the pericarp of table olives;

4. *Lactobacillus paracasei* deposited with the Belgian Coordinated Collections of Microorganism under accession number LMG P 22043.

## Patentansprüche

1. Tafeloliven, deren Perikarp mit probiotischen Lactobazillen behandelt wurde, gewonnen aus *Lactobacillus rhamnosus* und *L. paracasei,* und/oder deren Perikarp mit Bifidobakterien behandelt wurde, die aus *Bifidobacterium bifidum* und *B. longum* gewonnen wurden, mit der Eigenschaft, dass die Lactobazillen ausgewählt wurden aus: *Lactobacillus rhamnosus* GG ATCC53103; *L. rhamnosus* IMCP 11; *L. rhamnosus* IMCP 19; *Lactobacillus paracasei LMG P 22043; Lactobacillus paracasei* IMPC 4.1 und die Bifidobakterien aus *Bifidobacterium bifidum* ATCC 15696 and *Bifidobacterium longjpn M ATCC 15708;*

2. Tafeloliven nach Anspruch 1 mit der Eigenschaft, dass die Lactobazillen zum Stamm *Lactobacillus paracasei* gehören, wie in den Belgian Coordinated Collections of Microorganism unter der Zugangsnummer LMG P 22043 niedergelegt;

3. Der Einsatz von Lactobazillen und Bifidobakterien zur Beschichtung des Perikarps von Tafeloliven;

4. *Lactobacillus paracasei,* niedergelegt in den Belgian Coordinated Collections of Microorganism unter der Zugangsnummer LMG P 22043.

## Revendications

1. Les olives de table contenant des lactobacilles probiotiques, adhérant au péricarpe, sélectionnés parmi le groupe *Lactobacillus rhamnosus et L. paracasei* et/ou des bifidobactéries, adhérant au péricarpe, sélectionnées parmi le groupe *Bifidobacterium bifidum et B. longum* **caractérisées en ce que** les lactobacilles sont sélectionnés parmi le groupe : *Lactobacillus rhamnosus* GG ATCC53103 ; *L. rhamnosus* IMCP 11 ; *L. rhamnosus* IMCP 19 ; *Lactobacillus paracasei* LMG P 22043 ; *Lactobacillus paracasei* IMPC 4.1 et **en ce que** les bifidobactéries sont sélectionnées parmi le groupe : *Bifidobacterium bifidum* ATCC 15696 et *Bifidobacterium longum* ATCC 15708 ;

2. Les olives de table, selon la revendication 1, **caractérisées en ce que** les lactobacilles appartiennent à la souche de *Lactobacillus paracasei* déposée dans les Collections coordonnées belges de microorganismes sous le numéro d'accès LMG P 22043 ;

3. Utilisation de lactobacilles et de bifidobactéries pour couvrir le péricarpe des olives de table ;

4. *Lactobacillus paracasei* deposé dans les Collections coordonnées belges de microorganismes sous le numéro d'accès LMG P 22043.
